# EUROPEAN PATENT APPLICATION

(11) **EP 2 111 858 A1**
(43) Date of publication of application: **28.10.2009**
(21) Application number: 08155240.8
(22) Date of filing: 25.04.2008
(51) Int. Cl.: A61K 31/13, A61K 31/132, A61K 31/136, A61K 31/137, A61K 31/198, A61K 31/445, A61K 31/47, A61K 31/495, A61K 31/66, A61K 33/06, A61P 25/28

(54) **Novel treatment for alzheimer's disease**

(71) Applicant: EPFL Ecole Polytechnique Fédérale de Lausanne, 1015 Lausanne (CH)
(72) Inventor: The designation of the inventor has not yet been filed
(74) Representative: Schneiter, Sorin

(57) **Abstract**

The present invention concerns a new pharmaceutical composition comprising an antagonist and a co-agonist of the N-methyl-D-aspartate-type glutamate receptor (NMDAR). The inventors found that the co-administration of these two compounds effectively inhibits production of amyloide-β peptide. In patient's suffering from Alzheimer's disease, these peptides are deposited in the extracellular matrix forming neuritic "plaques". Therefore, the present inventors identified a new possibility for prophylaxis and/or treatment of Alzheimer's disease by stopping the continued formation of neuropathological extracellular deposits.

## Description

### Field of the Invention

The present invention relates to a pharmaceutical composition, in particular for the prophylaxis and/or treatment of Alzheimer's disease. The present invention also relates to a method of treatment of an individual suffering from Alzheimer's disease, in particular of a mild and/or early form of the disease, and to an inhibitor and a coagonist of the NMDA receptor in a combination therapy and/or prophylaxis of Alzheimer's disease.

### Background of the invention and Problems to be Solved

Alzheimer's disease (AD) is the most common form of neurodegenerative diseases in humans, leading to nerve cell death and tissue loss throughout the brain, therefore impairing memory, reasoning and behaviour and leading ultimately to complete social dependence and death. Unless effective strategies are discovered or developed to treat or prevent the progression of AD, these numbers are expected to rise significantly (25 million in the US by the year 2025), thus increasing the medical and economic burden on the individual families and society. Current therapeutic strategies for AD (listed below) offer mainly symptomatic and transient treatment and do not address the underlying cause of the disease.

During the past two decades, a working hypothesis known as the 'amyloid-β cascade hypothesis' has been developed suggesting that the progressive accumulation, oligomerization and/or aggregation of amyloid-β peptides (Aβ) in brain regions subserving memory and cognition, including the frontal cortex and the hippocampus, is the primary cause of neurodegeneration and cell death in Alzheimer's disease. Indeed, clinical, animal and cellular studies suggest a strong correlation between increased production, secretion and/or aggregation of Aβ and disease progression. Therefore, reducing Aβ levels can be considered as an important therapeutic strategy to prevent and/or treat Alzheimer's disease.

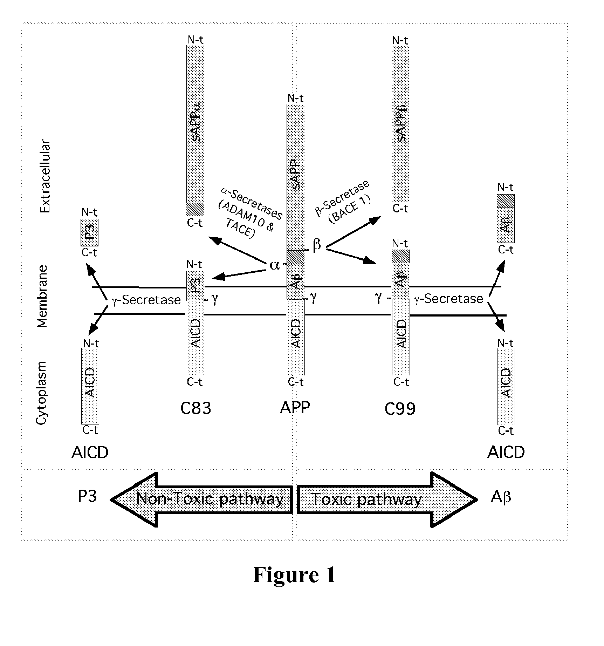

Aβ is formed from amyloid-β precursor protein (APP) through two protease activities. First, the membrane tethered aspartyl protease β-secretase (BACE 1) cleaves APP at the Aβ N-terminus (b cleavage site), resulting in a soluble form of APP (sAPPβ; Fig. 1) and a 99-residue (∼12kDa) C-terminal fragment (C99; Fig. 1). This C-terminal fragment undergoes subsequent processing by γ-secretase (γ-cleavage site) resulting in the formation of Aβ peptides of various lengths (39-42 amino-acid residues), with the two primary forms being the 40- and 42- amino acid variants, Aβ40 and Aβ42. Although Aβ42 accounts for only 10% of total Aβ secreted from cells (Aβ40 predominants), Aβ42 is the major protein component of amyloid plaques (the major pathological hallmark of AD) and aggregates much more rapidly than Aβ40 *in vitro.* An alternative non-amyloidogenic processing pathway of APP molecules involves its cleavage within the Aβ region (α cleavage site), by another member of the secretase family, the α-secretase metalloproteases ADAM 10 and tumor necrosis factor α (TNF-α)-converting enzyme (TACE/ADAM17). Cleavage by α-secretase precludes the formation of Aβ and results in the release of a soluble form of APP (sAPPα; Fig. 1) and membrane retention of an 83-residue (∼10kDa) C-terminal fragment (C83) (Fig. 1). Subsequent cleavage of C83 by γ-secretase results in an N-terminally truncated and non-toxic Aβ protein of 3 kDa (p3; Fig. 1). Further background information can be found in the publication of Patrick C. Fraering, Structural and functional determinants of γ-secretase, an intramembrane protease implicated in Alzheimer's disease. Current Genomics, 2007, Volume 8, No. 8.

Presently approved drugs for the treatment of Alzheimer's disease are (1) cholinesterase inhibitors (list) and (2) N-methyl-D-aspartate-type glutamate receptor (NMDAR) inhibitors such as memantine. Inhibition of cholinesterases, a family of acetylcholine-degrading enzymes, increases the synaptic concentration of the neurotransmitter acetylcholine, thereby enhancing and prolonging the action of acetylcholine on muscarinic and nicotinic acetylcholine receptors, with beneficial effects on cognition.

U.S. Pat. Nos. 6420351 and US 622875 disclose methods for treating neuropsychiatric disorders, such as schizophrenia, and teach a pharmaceutical composition comprising (i) an agonist of the glycine site of the N-methyl-D-aspartate (NMDA) receptor and (ii) a second therapeutic agent selected from a group also comprising Alzheimer's disease therapeutics. However, in these patents, no examples of combination treatments were actually made, thus not providing any concrete data. The only evidence shown relates to the treatment of schizophrenia patients with D-serine, D-alanine, and N-methylglycine. Furthermore, the few Alzheimer's disease therapeutics listed in this reference are cholinesterase inhibitors.

In view of the above it is an objective to provide a new prophylaxis and/or treatment for individuals suffering from Alzheimer's disease.

### Summary of the Invention

The present inventors found that the exposure of neurons to an NMDA receptor inhibitor and to an NMDA receptor coagonist effectively prevented the formation of the neurotoxic amyloid-β peptides. More particularly, exposure to a combination of these types of principles resulted in an activation of the non-amyloidogenic processing pathway of amyloid-β precursor proteins (APP), resulting in the generation of non-toxic soluble sAPPα.

Accordingly, the present invention provides, in a first aspect pharmaceutical composition comprising an inhibitor and a co-agonist of an N-methyl-D-aspartate-type glutamate receptor (NMDAR).

In a second aspect, the present invention provides an inhibitor and a co-agonist of the NMDAR for use as a medicament. Preferably, the two compounds are used in a combination prophylaxis and/or therapy. Accordingly, both compounds are administered in a way so as to be present in an individuals and/or patients serum in effective amounts.

In a third aspect, the present invention provides a method of preventing and/or treating Alzheimer's disease, the method comprising the step of administering to a person in need thereof a pharmaceutically effective amount of an inhibitor and a co-agonist of the N-methyl-D-aspartate-type glutamate receptor (NMDAR).

### Brief Description of the Drawings

**Figure 1** illustrates the processing of amyloid-β precursor protein (APP). APP is synthesized as a type I transmembrane protein which initially undergoes cleavage at either α- or β-secretase sites to release large ectodomains, designated sAPPα or sAPPβ, respectively, dividing the processing of APP into a toxic and a non-toxic pathway, respectively, as described above.

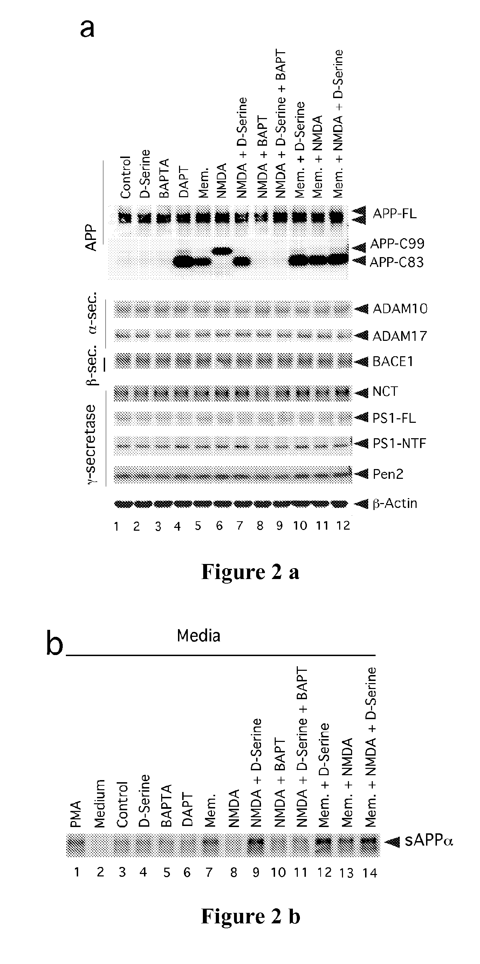

**Figure 2a** shows western blots of various protein and peptide components, including APP-C83 (the C-83 fragment of APP) and APP C-99 (the C-99 fragment of APP) isolated from rat primary hippocampal neurons following exposure to various modulators of the NMDA receptor, in particular, D-serine, memantine (Mem), and N-methyl-D-aspartate (NMDA). The presence of the C-83 fragment indicates that the non-amyloidogenic pathway via α-sectretase is active, whereas the C-99 fragment indicates that APP was cleaved by γ-secretase, giving way to the formation of neurotoxic Aβ. The figures show that co-administration of memantine and D-serine results in accumulation of APP-C83.
**Figure 2b** shows western blots of intracellular sAPPα secreted in media of cultured neurons unsed in Figure 1a. This figures mirrors the results of Figure 2a and shows that co-exposure to memantine and D-serine results in high amounts of sAPPα, an indicator for the non-toxic pathway.
**Figure 2c** shows the quantity of neurotoxic Aβ X-40 secreted by the neurons of Fig. 2a, as determined by ELISA. Cells exposed to memantine and D-serine show a large, significant decrease in the levels of Aβ X-40 when compared to those exposed to memantine or D-serine alone. A synergistic effect obtained by the co-administration is demonstrated in this figure.
**Figure 3a** shows the levels of intracellular APP-C99 and APP-C83 generated in the cells used in Fig. 2a, as estimated by densiometry (LI-COR odysses). This figure thus quantitatively shows the effects of NMDAR modulators on the dominance of one of the two APP processing pathways (Fig. 1) and again highlights the important preference of the non-toxic pathway in neurons exposed to memantine and D-serine.
**Figure 3b** shows a densiometric estimation as Fig. 3a, but here of secreted sAPPα, largely mirroring the results shown in Fig. 3a.
**Figure 4a** shows western blots as shown with respect to Figure 2a, with the difference that proteins and peptides were isolated from mouse primary hippocampal neurons.
**Figure 4b** shows western blots as shown with respect to Figure 2b, with the difference that sAPPα was isolated from mouse primary hippocampal neurons.
**Figure 5** shows a model for the NMDAR-dependent signaling pathways regulating β- and α-secretase cleavage of APP as hypothesised by the present inventors on the basis of their results.

### Detailed Description of the Preferred Embodiments

The present invention provides the administration of an inhibitor and of a coagonist of the NMDA receptor for the prophylaxis and/or the treatment of Alzheimer's disease.

For the purpose of the present specification, the term "inhibitor of the NMDAR" refers to a compound having the capacity of inhibiting or blocking to a measurable extent the activity of the NMDAR. Two types of inhibitors are particularly preferred and encompassed by the present invention: channel blockers and antagonists that do not bind in the channel of the NMDA receptor.

Presence or absence of an inhibiting effect of a compound on NMDA receptor channel activity can be determined by electrophysiological recording of recombinant channels by measuring steady-state currents after application of the compounds. For the purpose of the present specification, the methodology disclosed by Chen and Lipton "Pharmacological Implications of Two Distinct Mechanisms of Interaction of Memantine with N-Methyl-D-aspartate-Gated Channels", Pharmacol Exp Ther. 2005 Sep;314(3):961-71. Epub May 18, 2005 may be used to determine presence of an inhibitory effect. In this reference, the effect of memantine, administered in various concentrations, on steady-state currents is determined. According to the present invention, this methodology, using recombinant *Xenopus* oocytes, may be used to determine presence of an inhibiting effect of any candidate compound. If a compound administrated at a concentration of up to 1000 µM results in a fractional response of less than 100%, an inhibitory effect is found.

The terms "NMDA receptor" and "NMDAR", for the purpose of the present specification, refer to the so-called NMDA-subtype of a family of transmembrane receptors for the natural neurotransmitter glutamate. NMDAR are cation channels, which open upon binding of glutamate or NMDA. In general, NMDAR receptors are characterised by the presence of two NR1 and two NR2 subunits, all four units forming a channel in between them. Each pair of NR1 units and NR2 units may be identical or different, independently of each other. Preferably, the terms "NMDAR" or "NMDA receptor" refer to any receptor having two NR1 and two NR2 subunits. The terms "NMDAR" or "NMDA receptor" do not indicate any limitation to any specific isoform of the receptor, as found in specific brain areas and/or other tissues. For the purpose of determining the presence or absence of an inhibitory effect of a compound, the isoforms NR1-1a and NRA2A are preferably expressed in *Xenopus* oocytes as disclosed by Chen and Lipton (2005).

The inhibitor of the NMDA receptor according to the present invention may have a high, medium or low affinity for NMDAR, but medium or low affinity inhibitors are preferred.

According to a preferred embodiment, the inhibitor of the NMDA receptor is a NMDAR channel blocker. Accordingly, the inhibitor may be selected from compounds and compositions comprising one or more of the following compounds: Magnesium Mg²⁺, an 1-aminocyclohexane compound (including derivatives, such as memantine), phencyclidine, MK-801 ((+)-5-methyl-10,11-dihydro-5H-dibenzo[a,d]cyclohepten-5,10-imine maleate), ARL12495AA ((*S*)-phenyl-2-pyridine-ethanamine dihydrochloride) and ketamine (2-(2-chlorophenyl)-2-methylamino-cyclohexan-1-one), variants and/or derivatives of the aforementioned including those as described below, and combinations of two or more of the aforementioned.

According to a further embodiment, the inhibitor of the NMDA receptor is a competitive NMDA antagonist, such as one selected from CPP (3-(2-Carboxypiperazin-4-yl)propyl-1-phosphonic acid), AP5 (2-amino-5-phosphonovalerate), CGS19755 (cis-4-[Phosphomethyl]-piperidine-2-carboxylic acid), LY235959 (3S-(3a,4aa,6b,8aa)]-Decahydro-6-(phosphonomethyl)-3-is oquinolinecarboxylic acid), LY233053 ((2R*,4S*)-4-(1H-Tetrazol-5-ylmethyl)-2-piperidinecarboxylic acid), variants and/or derivatives of the aforementioned including those as described below, and combinations of two or more of the aforementioned.

According to an embodiment, the inhibitor of the NMDA receptor is a NR2B antagonist, such as one selected from Ro25-6981 ((aR,bS)-a-(4-Hydroxyphenyl)-b-methyl-4-(phenylmethyl)-1 -piperidinepropanol maleate), CP101606 (taxoprodil; ((1S,2S)-1-(4-hydroxyphenyl)-2-(4-hydroxy-4-phenylpiperidino)-1-propanol)), CI1041 (6-((2-(4-(4-fluorophenyl)methyl)-1-piperidinyl)ethyl)sulfinyl)-2-(3H)-benzoxazolone), ifenprodil ((1R*,2S*)-erythro-2-(4-Benzylpiperidino)-1-(4-hydroxyphenyl)-1-propanol), variants and/or derivatives of the aforementioned including those as described below, and combinations of two or more of the aforementioned.

The NMDAR inhibitor used for the purpose of the present invention may be selected among any one of the above listed channel blockers, competitive antagonists and NR2B antagonists. Furthermore, a combination comprising two or more compounds selected from the above categories of channel blockers, competitive antagonists and NR2B antagonists is also encompassed.

According to a preferred embodiment of the present invention, the NMDA receptor inhibitor is an aminocyclohexane compound, preferably a 1-aminocyclohexane compound.

The 1-aminocyclohexane compounds of the present invention having NMDA-channel blocking, inhibitory activity can be represented in the general formula (I): 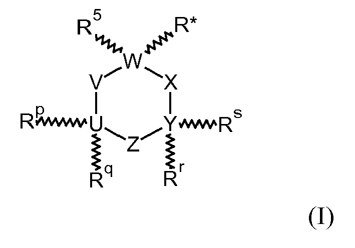 wherein:
R* is -(A)n-(CR¹R²)m-NR³R⁴,
   n and m are integers, and n+m=0, 1, or 2,
   A is selected from the group consisting of linear or branched lower alkyl (C1-C6), linear or branched lower alkenyl (C2-C6), and linear or branched lower alkynyl (C2-C6);
   R¹ and R² are independently selected from the group consisting of hydrogen, linear or branched lower alkyl (C1-C6), linear or branched lower alkenyl (C2-C6), linear or branched lower alkynyl (C2-C6) aryl, substituted aryl and arylalkyl;
   R³ and R⁴ are independently selected from the group consisting of hydrogen, linear or branched lower alkyl (C1-C6), linear or branched lower alkenyl (C2-C6), and linear or branched lower alkynyl (C2-C6), or together form alkylene (C2-C10) or alkenylene (C2-C10) or together with the N form a 3-7-membered azacycloalkane or azacycloalkene, including a substituted (alkyl (C1-C6), alkenyl (C2-C6)) 3-7-membered azacycloalkane or azacycloalkene; or independently R³ or R⁴ may join with R^{p}, R^{q}, R^{r}, or R^{s} to form an alkylene chain -CH(R⁶)-(CH₂)t-, wherein t=0 or 1 and the left side of the alkylene chain is attached to U or Y and the right side of the alkylene chain is attached to N and R⁶ is selected from the group consisting of hydrogen, linear or branched lower alkyl (C1-C6), linear or branched lower alkenyl (C2-C6), linear or branched lower alkynyl (C2-C6), aryl, substituted aryl and arylalkyl; or independently R³ or R⁴ may join with R⁵ to form an alkylene chain represented by the formula -CH2-CH2-CH2-(CH2)t-, or an alkenylene chain represented by the formulae -CH-CH-CH2-(CH2)t-, -CH-C-CH-(CH2)t- or -CH2-CH-CH-(CH₂)ₜ-, wherein t=0 or 1, and the left side of the alkylene or alkenylene chain is attached to W and the right side of the alkylene ring is attached to N;
R⁵ is independently selected from the group consisting of hydrogen, linear or branched lower alkyl (C1-C6), linear or branched lower alkenyl (C2-C6), and linear or branched lower alkynyl (C2-C6), or R⁵ combines with the carbon to which it is attached and the next adjacent ring carbon to form a double bond;
R^{p}, R^{q}, R^{r}, and R^{s}, are independently selected from the group consisting of hydrogen, linear or branched lower alkyl (C1-C6), linear or branched lower alkenyl (C2-C6), linear or branched lower alkynyl (C2-C6), cycloalkyl (C3-C6) and aryl, substituted aryl and arylalkyl or R^{p}, R^{q}, R^{r}, and R^{s} independently may form a double bond with U or with Y or to which it is attached, or R^{p}, R^{q}, R^{r}, and R^{s} may combine together to represent a lower alkylene -(CH₂)ₓ-or a lower alkenylene bridge wherein x is 2-5, inclusive, which alkylene bridge may, in turn, combine with R⁵ to form an additional lower alkylene -(CH₂)_{y}- or a lower alkenylene bridge, wherein y is 1-3, inclusive; and
the ring defined by U-V-W-X-Y-Z represents an optionally unsaturated cyclohexane ring wherein U, W, and Y represent carbon atoms and V, X, and Z each independently represent a carbon atom, CH, or CH₂, (or the definitions of U, W, Y on one hand and V, X, and Z can be reversed including corresponding placement of the R groups R*, R⁵, R^{p}, R^{q}, R^{r}, and R^{s}), it being understood that the valence requirements of the ring atoms are respected, and include optical isomers, diastereomers, polymorphs, enantiomers, hydrates, pharmaceutically acceptable salts, and mixtures of compounds within formula (I).

The ring defined by U-V-W-X-Y-Z is preferably selected from the group consisting of cyclohexane, cyclohex-2-ene, cyclohex-3-ene, cyclohex-1,4-diene, cyclohex-1,5-diene, cyclohex-2,4-diene, and cyclohex-2,5-diene.

Compounds of formula I may be adamantyl substances.

Non-limiting examples of 1-aminocyclohexane derivatives used according to the invention include the 1-aminoalkylcyclohexane derivatives selected from the group consisting of:
1-amino-1,3,5-trimethylcyclohexane,
1-amino-1 (trans),3(trans),5-trimethylcyclohexane,
1-amino-1 (cis),3(cis),5-trimethylcyclohexane,
1-amino-1,3,3,5-tetramethylcyclohexane,
1-amino-1,3,3,5,5-pentamethylcyclohexane (neramexane),
1-amino-1,3,5,5-tetramethyl-3-ethylcyclohexane,
1-amino-1,5,5-trimethyl-3,3-diethylcyclohexane,
1-amino-1,5,5-trimethyl-cis-3-ethylcyclohexane,
1-amino-(1S,5S)cis-3-ethyl-1,5,5-trimethylcyclohexane,
1-amino-1,5,5-trimethyl-trans-3-ethylcyclohexane,
1-amino-(1R,5S)trans-3-ethyl-1,5,5-trimethylcyclohexane,
1-amino-1-ethyl-3,3,5,5-tetramethylcyclohexane,
1-amino-1-propyl-3,3,5,5-tetramethylcyclohexane,
N-methyl-1-amino-1,3,3,5,5-pentamethylcyclohexane,
N-ethyl-1-amino-1,3,3,5,5-pentamethyl-cyclohexane,
N-(1,3,3,5,5-pentamethylcyclohexyl) pyrrolidine,
3,3,5,5-tetramethylcyclohexylmethylamine,
1-amino-1-propyl-3,3,5,5-tetramethylcyclohexane,
1 amino-1,3,3,5(trans)-tetramethylcyclohexane (axial amino group),
3-propyl-1,3,5,5-tetramethylcyclohexylamine semihydrate,
1-amino-1,3,5,5-tetramethyl-3-ethylcyclohexane,
1-amino-1,3,5-trimethylcyclohexane,
1-amino-1,3-dimethyl-3-propylcyclohexane,
1-amino-1,3(trans),5(trans)-trimethyl-3 (cis)-propylcyclohexane,
1-amino-1,3-dimethyl-3-ethylcyclohexane,
1-amino-1,3,3-trimethylcyclohexane,
cis-3-ethyl-1(trans)-3((trans)-5-trimethylcyclohexamine,
1-amino-1,3 (trans)-dimethylcyclohexane,
1,3,3-trimethyl-5,5-dipropylcyclohexylamine,
1-ammo-1-methyl-3 (trans)-propylcyclohexane,
1-methyl-3(cis)-propylcyclohexylamine,
1-amino-1-methyl-3 (trans)-ethylcyclohexane,
1-amino-1,3,3-trimethyl-5(cis)-ethylcyclohexane,
1-amino-1,3,3-trimethyl-5(trans)-ethylcyclohexane,
cis-3-propyl-1,5,5-trimethylcyclohexylamine,
trans-3-propyl-1,5,5-trimethylcyclohexylamine,
N-ethyl-1,3,3,5,5-pentamethylcyclohexylamine,
N-methyl-1-amino-1,3,3,5,5-pentamethylcyclohexane,
1-amino-1-methylcyclohexane,
N,N-dimethyl-1-amino-1,3,3,5,5-pentamethylcyclohexane,
2-(3,3,5,5-tetramethylcyclohexyl)ethylamine,
2-methyl-1-(3,3,5,5-tetramethylcyclohexyl)propyl-2-amine,
2-(1,3,3,5,5-pentamethylcyclohexyl-1)-ethylamine semihydrate,
N-(1,3,3,5,5-pentamethylcyclohexyl)-pyrrolidine,
1-amino-1,3(trans),5(trans)-trimethylcyclohexane,
1-amino-1,3(cis),5(cis)-trimethylcyclohexane,
1-amino-(1R,SS)trans-5-ethyl-1,3,3-trimethylcyclohexane,
1-amino-(1S,SS)cis-5-ethyl-1,3,3-trimethylcyclohexane,
1-amino-1,5,5-trimethyl-3(cis)-isopropyl-cyclohexane,
1-amino-1,5,5-trimethyl-3(trans)-isopropyl-cyclohexane,
1-amino-1-methyl-3(cis)-ethyl-cyclohexane,
1-amino-1-methyl-3(cis)-methyl-cyclohexane,
1-amino-5,5-diethyl-1,3,3-trimethyl-cyclohexane,
1-amino-1,3,3,5,5-pentamethylcyclohexane,
1-amino-1,5,5-trimethyl-3,3-diethylcyclohexane,
1-amino-1-ethyl-3,3,5,5-tetramethylcyclohexane,
N-ethyl-1-amino-1,3,3,5,5-pentamethylcyclohexane,
N-(1,3,5-trimethylcyclohexyl)pyrrolidine or piperidine,
N-[1,3(trans),5(trans)-trimethylcyclohexyl]pyrrolidine or piperidine,
N-[1,3(cis),5(cis)-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,3,3-trimethyl-cis-5-ethylcyclohexyl)pyrrolidine or piperidine,
N-[(1S,SS)cis-5-ethyl-1,3,3-trimethylcyclohexyl]pyrrolidine or piperidine,
N-(1,3,3-trimethyl-trans-5-ethylcyclohexyl)pyrrolidine or piperidine,
N-[(1R,SS)trans-5-ethy1,3,3-trimethylcyclohexyl]pyrrolidine or piperidine,
N-(1-ethyl-3,3,5,5-tetramethylyclohexyl)pyrrolidine or piperidine,
N-(1-propyl-3,3,5,5-tetramethylcyclohexyl)pyrrolidine or piperidine,
N-(1,3,3,5,5-pentamethylcyclohexyl)pyrrolidine,
their optical isomers, diastereomers, enantiomers, hydrates, their pharmaceutically acceptable salts, and mixtures thereof.

A further preferred compound for the composition of the invention is neramexane (1-amino-1,3,3,5,5-pentamethylcyclohexane), which is disclosed, e.g., in U.S. Pat. No. 6034134, which is incorporated herein by reference in its entirety.

Formulae IIa-IId bellow illustrate certain 1-aminocyclohexane derivatives of general formula (I) according to the present invention, including the case where three axial alkyl substituent, e.g., R^{p}, R^{r} and R⁵ all together form a bridgehead to yield compounds (so called 1-aminoadamantanes) (formulae IIb and IId) below: 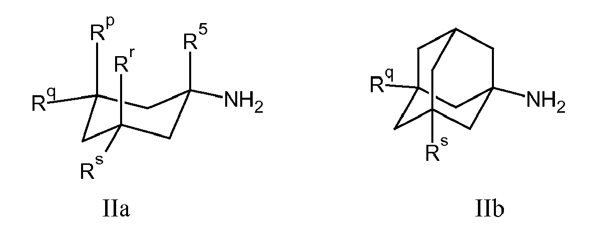 or

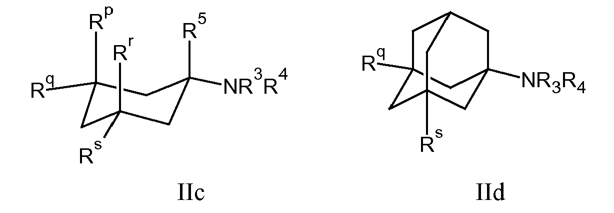

Certain 1-aminocyclohexane derivatives of formula (I) wherein n+m=0, U, V, W, X, Y and Z form a cyclohexane ring, and one or both of R³ and R⁴ are independently joined to said cyclohexane ring via alkylene bridges formed through R^{p}, R^{q}, R^{r}, R^{s} or R⁵ are represented by the following formulae IIIa-IIIc: 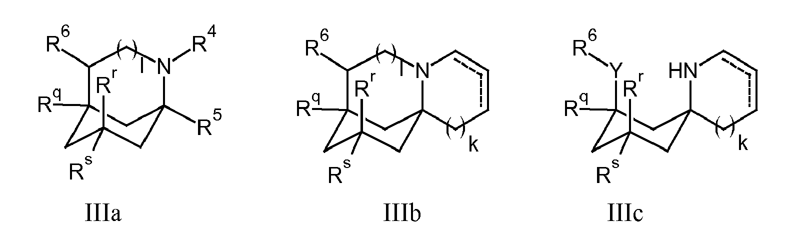 wherein R^{q}, R^{r}, R^{s}, R^{r} and R⁵ are as defined above for formula (I), R⁶ is hydrogen, linear or branched lower alkyl (C1-C6), linear or branched lower alkenyl (C2-C6), linear or branched lower alkynyl (C2-C6), aryl, substituted aryl or arylalkyl, Y is saturated or may combine with R⁶ to form a carbon-hydrogen bond with the ring carbon to which it is attached, 1=0 or 1 and k=0, 1 or 2 and represents a single or double bond.

Non-limiting examples of 1-aminocyclohexane derivatives used according to the invention include 1-amino adamantane and its derivatives selected from the group consisting of:
1-amino-3-phenyl adamantane,
1-amino-methyl adamantane,
1-amino-3,5-dimethyl adamantane (memantine),
1-amino-3-ethyl adamantane,
1-amino-3-isopropyl adamantane,
1-amino-3-n-butyl adamantane,
1-amino-3,5-diethyl adamantane,
1-amino-3,5-diisopropyl adamantane,
1-amino-3,5-di-n-butyl adamantane,
1-amino-3-methyl-5-ethyl adamantane,
1-N-methylamino-3,5-dimethyl adamantane,
1-N-ethylamino-3,5-dimethyl adamantane,
1-N-isopropyl-amino-3,5-dimethyl adamantane,
1-N,N-dimethyl-amino-3,5-dimethyl adamantane,
1-N-methyl-N-isopropyl-amino-3-methyl-5-ethyl adamantane,
1-amino-3-butyl-5-phenyl adamantane,
1-amino-3-pentyl adamantane,
1-amino-3,5-dipentyl adamantane,
1-amino-3-pentyl-5-hexyl adamantane,
1-amino-3-pentyl-5-cyclohexyl adamantane,
1-amino-3-pentyl-5-phenyl adamantane,
1-amino-3-hexyl adamantane,
1-amino-3,5-dihexyl adamantane,
1-amino-3-hexyl-5-cyclohexyl adamantane,
1-amino-3-hexyl-5-phenyl adamantane,
1-amino-3-cyclohexyl adamantane,
1-amino-3,5-dicyclohexyl adamantane,
1-amino-3-cyclohexyl-5-phenyl adamantane,
1-amino-3,5-diphenyl adamantane,
1-amino-3,5,7-trimethyl adamantane,
1-amino-3,5-dimethyl-7-ethyl adamantane,
1-amino-3,5-diethyl-7-methyl adamantane,
1-N-pyrrolidino and 1-N-piperidine derivatives,
1-amino-3-methyl-5-propyl adamantane,
1-amino-3-methyl-5-butyl adamantane,
1-amino-3-methyl-5-pentyl adamantane,
1-amino-3-methyl-5-hexyl adamantane,
1-amino-3-methyl-5-cyclohexyl adamantane,
1-amino-3-methyl-5-phenyl adamantane,
1-amino-3-ethyl-5-propyl adamantane,
1-amino-3-ethyl-5-butyl adamantane,
1-amino-3-ethyl-5-pentyl adamantane,
1-amino-3-ethyl-5-hexyl adamantane,
1-amino-3-ethyl-5-cyclohexyl adamantane,
1-amino-3-ethyl-5-phenyl adamantane,
1-amino-3-propyl-5-butyl adamantane,
1-amino-3-propyl-5-pentyl adamantane,
1-amino-3-propyl-5-hexyl adamantane,
1-amino-3-propyl-5-cyclohexyl adamantane,
1-amino-3-propyl-5-phenyl adamantane,
1-amino-3-butyl-5-pentyl adamantane,
1-amino-3-butyl-5-hexyl adamantane,
1-amino-3-butyl-5-cyclohexyl adamantane,
their optical isomers, diastereomers, enantiomers, hydrates, N-methyl, N,N-dimethyl, N-ethyl, N-propyl derivatives, their pharmaceutically acceptable salts, and mixtures thereof.

Memantine (1-amino-3,5-dimethyl adamantane), for example, is the subject matter of U.S. Pat. Nos. 4,122,193 and 4,273,774, both incorporated herein by reference in their entirety.

The 1-amino adamantane compounds of formulae IIb and IId, including memantine, are generally prepared by alkylation of halogenated adamantanes, preferably bromo- or chloroadamantanes. The di- or tri-substituted adamantanes are obtained by additional halogenation and alkylation procedures. The amino group is introduced either by oxidation with chromiumtrioxide and bromination with HBr or bromination with bromine and reaction with formamide followed by hydrolysis. The amino function can be alkylated according to generally-accepted methods. Methylation can, for example, be effected by reaction with chloromethyl formate and subsequent reduction. The ethyl group can be introduced by reduction of the respective acetamide. For more details on synthesis see, e.g., U.S. Pat. Nos. 5,061,703 and 6,034,134. Additional synthetic techniques for the foregoing compounds can be found in published U.S. Application Nos. 2003/0166634 and 2004/0034055, all incorporated by reference in their entirety.

According to the invention, the NMDAR inhibitors disclosed herein, such as the 1-aminocyclohexane derivatives of formula (I), may be applied as such or used in the form of their pharmaceutically acceptable salts. Suitable salts of the compound include, but are not limited to, acid addition salts, such as those made with hydrochloric, methylsulfonic, hydrobromic, hydroiodic, perchloric, sulfuric, nitric, phosphoric, acetic, propionic, glycolic, lactic pyruvic, malonic, succinic, maleic, fumaric, maleic, tartaric, citric, benzoic, carbonic cinnamic, mandelic, methanesulfonic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid; salts made with saccharin. In a preferred embodiment, the salt is memantine hydrochloride (C₁₂H₂₁N·HCl, MW 215.77). In another preferred embodiment, the salt is neramexane mesylate (C₁₁H₂₃N·CH₄O₃S, MW 265.42). The term "salts" can also include addition salts of free acids. All of these salts (or other similar salts) may be prepared by conventional means. All such salts are acceptable provided that they are non-toxic and do not substantially interfere with the desired pharmacological activity.

The present invention further includes all individual enantiomers, diastereomers, racemates, and other isomers of those NMDAR inhibitors and/or coagonists wherein such structural variations are possible. The invention also includes all polymorphs and solvates, such as hydrates and those formed with organic solvents, of these compounds. Such isomers, polymorphs, and solvates may be prepared by methods known in the art, such as by crystallization from different solvents, or by regiospecific and/or enantioselective synthesis and resolution, based on the disclosure provided herein.

The present invention includes further derivatives of the 1-aminocyclohexane compounds of the present invention. Examples of derivatives applicable to the invention include, but are not limited to, structurally related compounds composed of a tricyclic 10-carbon ring bearing an amino group such as nitroxy-memantine derivatives (such as nitroprusside, nitroglycerin, or an NO-generating derivative of nitroprusside or nitroglycerin in U.S. Pat. Nos. 5,234,956 and 5,455,279).

In one preferred embodiment, the active ingredient is memantine hydrochloride. In another preferred embodiment, the active ingredient is neramexane mesylate.

For the purpose of the present invention, the term "co-agonist" or "coagonist" of the NMDAR" preferably refers to a compound binding to the glycine binding site of the NMDAR.

Specific examples of such co-agonists are disclosed in U.S. Pat. No. 6420.351, where they are designated as "agonist of the glycine site of an NMDA receptor". They include D-alanine, D-cycloserine, D-serine, N-methylglycine, D-phosphoserine, L-phosphoserine, but also glycine, and any combination of two or more of the aforementioned.

According to the invention, glycine D-alanine, D-serine, and/or D-cycloserine and/or N-methylglycine may be substituted with a modified version of the amino acid, such as a salt, ester, alkylated form, or a precursor of the amino acid. For example, the amino acid can be in the form of a sodium salt, potassium salt, calcium salt, magnesium salt, zinc salt, or ammonium salt. Such salt forms of D-serine, D-alanine, N-methylglycine and D-cycloserine can be made in accordance with conventional methods (see, e.g., Organic Chemistry, pgs. 822-823, Morrison and Boyd, ed., Fifth Edition, Allyn and Bacon, Inc., Newton, Mass.). Other modified forms of D-serine, D-alanine, N-methylglycine and D-cycloserine also can be used in the methods of the invention. For example, the carboxy group of the amino acid can be converted to an ester group by reaction with an alcohol in accordance with standard esterification methods (Id. at 841-843). For example, alcohols having 1-20 carbon atoms can be used to produce an ester of D-serine, D-alanine, N-methylglycine or D-cycloserine for use in the invention (e.g., methyl-, ethyl-, propyl-, isopropyl-, butyl-, isobutyl-, sec-butyl-, tert-butyl-, pentyl-, isopentyl-, tert-pentyl-, hexyl-, heptyl-, octyl-, decyl-, dodecyl-, tetradecyl-, hexadecyl-, octadecyl-, and phenyl-alcohols can be used). In another variation, the amino group of the amino acid can be alkylated, using conventional methods, to produce a secondary or tertiary amino group by ammonolysis of halides or reductive amination (Id. at 939-948). For example, an alkyl group having 1-20 carbon atoms can be added to the amino acid to produce an alkylated amino acid (e.g., methyl-, ethyl-, propyl-, isopropyl-, butyl-, isobutyl-, sec-butyl-, tert-butyl-, pentyl-, isopentyl-, tert-pentyl-, hexyl-, heptyl-, octyl-, decyl-, dodecyl-, tetradecyl-, hexadecyl-, octadecyl- and phenyl-groups can be added to the amino acid). D-phosphoserine and L-phosphoserine are examples of precursors of D-serine, and are commercially available (e.g., from Sigma Chemical, St. Louis, Mo.). N,N,N-trimethylglycine (betaine) and N,N-dimethylglycine are examples of precursors of N-methylglycine.

According to the invention, appropriate dosages of the NMDAR inhibitor and the coagonist can readily be determined by those of ordinary skill in the art of medicine by monitoring the patient for signs of disease amelioration or inhibition, and increasing or decreasing the dosage and/or frequency of treatment as desired.

The pharmaceutical compositions of the invention can be administered to the patient by any, or a combination, of several routes, such as oral, intravenous, trans-mucosal (e.g., nasal, vaginal, etc.), pulmonary, transdermal, ocular, buccal, sublingual, intraperitoneal, intrathecal, intramuscular, or long term depot preparation. Solid compositions for oral administration can contain suitable carriers or excipients, such as corn starch, gelatin, lactose, acacia, sucrose, microcrystalline cellulose, kaolin, mannitol, dicalcium phosphate, calcium carbonate, sodium chloride, lipids, alginic acid, or ingredients for controlled slow release. Disintegrators that can be used include, without limitation, micro-crystalline cellulose, corn starch, sodium starch glycolate and alginic acid. Tablet binders that may be used include, without limitation, acacia, methylcellulose, sodium carboxymethylcellulose, polyvinylpyrrolidone (Povidone), hydroxypropyl methylcellulose, sucrose, starch, and ethylcellulose.

Importantly, the effective and synergistic combination of at least two compounds, an NMDAR inhibitor and an NMDAR coagonist may each be provided in a separate administration unit, the term "administration unit" referring to a tablet, pills, capsules, solutions, and so forth, that is suitable for administration. Accordingly, each administration unit may contain only one of the two compounds. In this case, an individual is required to be administered two different administration units. The two administration units may be administered simultaneously or sequentially, with a relatively short time period between the two administration steps, so as to ensure that an effective simultaneous concentration of each compound in the individual is achieved. If the two compounds are administered separately in the form of separate administration units, it is also possible to use a separate administration route for each of the two compounds of the composition of the present invention, for example one compound may be orally administered and the other intravenuously.

Alternatively, the composition may be administered in the literal form of the term "composition", meaning a mixture of the two compounds, for example in the form of a single tablet, pill, etc or a solution or suspension comprising both compounds, which is administered in a single administration step.

The examples below illustrate the invention and are do not limit its scope.

### Example

### Effect of NDMA, NMDAR co-agonist D-serine, and/or antagonist memantine on α- and β-secretase cleavage of APP

In the experiments given below, the following abbreviations may be used:
Aβ, amyloid-β protein; AD, Alzheimer's disease; APP, amyloid-β precursor protein; AICD, APP intracellular domain; CHO, Chinese hamster ovary; CTF, C-terminal fragment; ER, endoplasmic reticulum; FAD, familial Alzheimer's disease; GST, glutathione-S-transferase; GSH, glutathione; HMW, high molecular weight; IP, immunoprecipitation; kDa, kiloDalton; NCT, Nicastrin; NTF, N-terminal fragment; PC, phosphatidylcholine; PE, phosphatidylethanolamine; PS, presenilin; SR, serine racemase, TM, transmembrane; TMD, transmembrane domain; WT, wild-type.

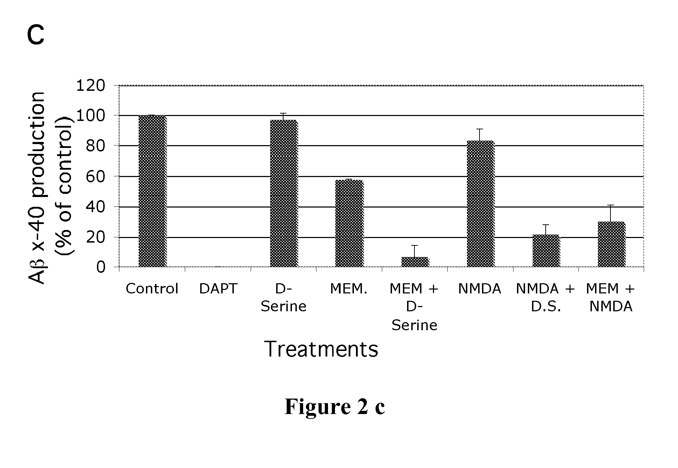

As a first step to examine the role of NMDA receptor agonists and antagonists on α-, β- and γ-secretase cleavage of APP, rat primary hippocampal neurons were exposed to the following compounds: D-serine, 1,2-bis(o-aminophenoxy)ethane-N,N,N',N'-tetraacetic acid (BAPTA), *N*-[N-(3,5-difluorophenacetyl)-L-alanyl]-S-phenylglycine t-butyl ester (DAPT), 1-amino-3,5-dimethyladamantane (memantine, Mem), N-methyl-D-aspartate (NMDA), and combinations of these compounds as indicated in Fig. 2, and the intracellular accumulation of γ-secretase substrates APP-C99 and APP-C83 were probed by western blot.

When compared to the untreated control (Fig. 2a, lane 1), memantine, alone or in combination with D-serine (Mem. + D-serine), NMDA (Mem. + NMDA) or NMDA + D-serine (Mem. + NMDA + D-Serine), triggered the accumulation of APP-C83 (Fig. 2a, lanes 5, 10, 11 & 12, respectively).

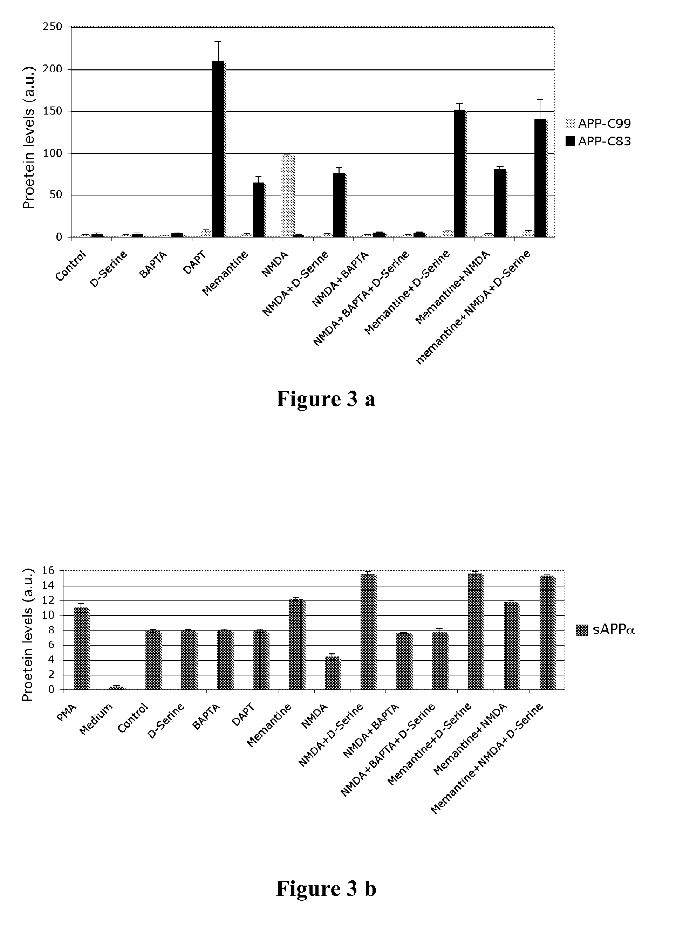

D-serine by itself did not affect the production of APP-C99/APP-C83 by β-secretase and α-secretase, respectively (Fig. 2a, lane 2). Nevertheless, D-serine, when combined to memantine, substantially increased the levels of APP-C83, when compared to memantine alone (Fig. 2a, compare APP-C83 levels in lanes 10 and 5). Incubation with DAPT, a well-known inhibitor of γ-secretase resulted in increased levels of both APP-C83 and APP-C99, with APP-C83 found in large excess when compared to APP-C99 (Fig. 2a, lane 4). Quantitative analysis revealed an APP-C83/APP-C99 ratio of 1:30 (Fig. 3a). Because APP is a limiting substrate for which both α- and β-secretase activities compete, the levels of APP-C83 and APP-C99 observed after DAPT treatment strongly suggest that the non-amyloidogenic pathway (α-secretase pathway) is predominant in control neurons cultured under these experimental conditions. Based on this observation, and in sharp contrast to the effects observed in cells incubated with memantine, NMDA treatment of rat primary cortical neurons resulted in the intracellular accumulation of APP-C99 (Fig. 2a, lane 6). Intriguingly, a combination of NMDA and D-serine resulted in the exclusive accumulation of APP-C83 (Fig. 2a, compare lane 7 with lane 6). This observation strongly suggests that D-serine, when combined to NMDA, affects the competition between α-versus β-secretase for APP, resulting in increased α-secretase cleavage of APP at the expense of β-secretase.

To support our hypothesis, sAPPα secretion (reflecting directly α- and β-secretase cleavage of APP) was examined in the corresponding conditioned media. Phorbol 12-myristate 13-acetate (PMA), a PKC activator that has been reported to stimulate α-secretase cleavage of APP while reducing β-secretase¹³, was used as a control (Fig. 2b, lane 1). Fully consistent with increased α-secretase cleavage of APP, secretion of sAPPα was increased by 1.5 to 2-fold following memantine-based or NMDA+D-serine treatments (Fig.2b, compare sAPPα levels in lanes 7, 9, 12, 13 & 14 with control lane 3 - quantitative analysis in Fig. 3b). Yet, secretion of sAPPα was reduced following NMDA treatment (Fig. 2b, compare sAPPα levels in lane 8 with control lane 3), consistent with reduced α-secretase cleavage of APP. When taken together, changes seen in sAPPα levels mirrored with changes seen in APP-C83 and APP-C99 levels.

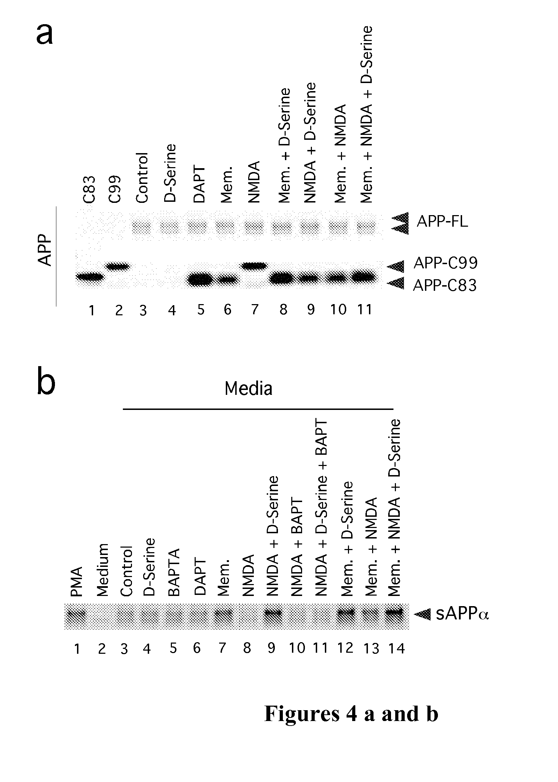

To know whether the treatments described above influence the levels of secreted Aβ, ELISA analyses were performed. Consistent with APP-C99/C83 levels, APP-C83-accumulating treatments lowered AβX-40 (Fig. 2c). Furthermore, D-Serine, when combined to memantine, substantially lowered AβX-40, when compared to memantine alone (Fig. 2c). Similar data were found for Aβ1-40 and Aβ1-42. Of note, none of the treatments did cause neuronal cell death. Furthermore, and as shown in Fig. 2a, the levels of APP full-length (APP-FL), γ-secretase (the subunits NCT, PS1-FL, PS1-NTF and Pen-2 are shown), ADAM10 and ADAM17 (responsible for α-secretase activity) and BACE1 (responsible for β-secretase activity) were unaffected by the different treatments. This argues against an alteration in the protein levels of the different secretases involved in the proteolytic processing of APP, but supports a mechanism that is responsible for regulating the enzymatic activities involved in the non-amyloidogenic and amyloidogenic pathways. Supporting our findings, very similar data were found in mice hippocampal neurons (Fig. 4).

### Conclusions

The above experiments show that the combined administration of memantine, a NMDA receptor antagonist, and D-serine, a coagonist of the NMDA receptor, are suitable to prevent the formation of amyloid-β peptide (Aβ) found in neuritic "plaques" in the brain of individuals suffering from Alzheimer's disease. The combination of a NMDA receptor antagonist and coagonist thus provide a new treatment and/or prophylaxis for Alzheimer's disease.

In general, in view of the results reported herein, supplementing D-serine to memantine or other NMADR inhibitor is expected to result in synergistic therapeutic benefits, especially in individuals with early to mild Alzheimer's disease (persons with clinical signs of cognitive impairment) or in individuals who may be at risk of developing this disease, for example due to harbouring clinical mutations in APP or PS1/PS2, or being heterozygous/homozygous in Apolipoprotein E isoform 4.

### Methods

Neuronal cultures and treatments. Cortical and hippocampal neurons were cultured as described²⁷ from postnatal day 0 rats or mice and all treatments described bellow carried out at 15 days *in vitro* (DIV). Stimulations with 30µM N-methyl-D-aspartate (NMDA, Tocris), 30µM D-serine (the D isoform of the amino acid serine, Sigma), or a combination of NMDA + D-serine (30µM each) were carried out for 3 minutes and neurons transferred into trophically enriched medium and further incubated for 16 hours. Treatments with 30µM 1-amino-3,5-dimethyladamantane (memantine, Lucerna-Chem), 1 µM N-[N-(3,5-difluorophenacetyl)-L-alanyl]-S-phenylgly cine t-butyl ester (DAPT, ), and 1 µM phorbol 12-myristate 13-acetate (PMA, Sigma) were carried out for 16 hours. Pre-treatments with 1 µM TAPI-1 (Calbiochem), 1 µM GL189 (Calbiochem), 1,2-bis(o-aminophenoxy)ethane-N,N,N',N'-tetraacetic acid (BAPTA, Sigma), H-89, chelerythrine, or KN-62 (Biaffin GmbH & Co KG) were carried out for 1 hour. Next, neurons were treated (or not) with NMDA or NMDA + D-serine as described above, and cells transferred into trophically enriched medium and incubated for additional 15 hours. Whole cell lysates were prepared for Western blot analysis as described below.

Western blotting and antibodies. Cells were washed with cold phosphate-bufferd saline containing 2 mM EDTA and homogenized in 50 mM Tris-HCL pH 7.4, 350 mM NaCl, 100 mM DTT, 0.1% SDS, 0.5% NP-40 and a cocktail of protease inhibitors (Roche Diagnostics). Protein whole cell extracts were separated on 4-12% Bis-tris gels (Invitrogen) and transferred onto nitrocellulose membranes to detect APP-FL, APP-C99, APP-C83 and AICD (CT15, Sigma), sAPPα (6E10, Signet), Notch-FL and NICD (Sigma), ADAM10 (Calbiochem), ADAM17 (Calbiochem), BACE1 (EE17, Sigma), NCT (NCT164, BD Bioscience), PS1-FL and PS1-NTF (MAB1536, Chemicon International), Pen-2 (UD-1, 1:500, a gift of Helena Karlström), and β-Action (Sigma).

Animals. OF1 mice and Wistar rats were provided by Charles River and Janvier, respectively. We carried out all animal experiments in accordance with the local directive for care and use of laboratory animals.

Immunohistochemistry. Cryostat sections of 16µm thickness were incubated with primary antibodies diluted in 4% bovine serum albumin, 5% donkey serum in PBS containing 0.1% Triton-X100. Immunoreactivity was visualized with fluorochrome-conjugated secondary antibodies (Jackson Laboratories).

Aβ1-40 and Aβ1-42 quantitative assay. Aβ1-40 and Aβ1-42 peptides secreted in the conditioned media were quantitatively measured by ELISA (Invitrogen) according to the protocol provided by the manufacturer.

### Detailed Legend of the Figures

Fig. 1. APP processing and the production of the amyloid-β peptides. APP is synthesized as a type I transmembrane protein which initially undergoes cleavage at either α- or β-secretase sites to release large ectodomains, designated sAPPα or sAPPβ, respectively. Ectodomain shedding leaves membrane-embedded fragments, C99 (99-residue C-terminal APP fragment) or C83 (83-residue C-terminal APP fragment), which are substrates for γ-secretase cleavage. Proteolysis of C99 by γ-secretase promotes the extracellular release of the amyloidogenic fragments (Aβ, toxic pathway) whereas proteolysis of C83 by γ-secretase generates an N-terminally truncated and non- toxic Aβ protein of 3 kDa (P3, non-toxic pathway). Evidence suggests that the progressive accumulation/oligomerization/aggregation of Aβ in brain regions subserving memory and cognition is the primary cause of neurodegeneration in Alzheimer's disease. Proteolysis of C99 and C83 by γ-secretase also releases the APP intracellular domain (AICD). Following association with the adaptor protein Fe65 and nuclear translocation, AICD is able to suppress the expression of the major apoE/lipoprotein receptor LRP1 by binding directly to its promoter. Thus, APP processing is also involved in the regulation of brain apoE (a major genetic determinant of AD) and cholesterol metabolism.
Fig. 2. NMDAR modulators discriminate between the Ca2+-dependent production of APP-C99 and APP-C83. (a) Western blot analysis of intracellular APP full length (APP-FL) and APP-C-terminal fragments C99 (APP-C99) and C83 (APP-C83) generated in rat primary cortical neurons (15 days *in vitro*) exposed to NMDAR agonist NMDA (30µM, for 3 mins), co-agonist D-Serine (30 µM, for 3 mins), or antagonist Memantine (30µM, for 16 hours), in the presence or absence of Ca2+ chelating reagent BAPTA (1 µM, pre-treatment for 1 hour). Treatments were carried out for the times indicated above and neurons were transferred into trophically rich medium as described in Methods. The corresponding cell lysates were blotted for α-secretases ADAM10 and ADAM17, β-secretase Brace-1, and γ-secretase components NCT, PS1 holoprotein (PS1-FL), PS1-NTF, and Pen-2. (b) sAPPα secreted in conditioned media of cultured neurons used in (a) were analyzed by Western blot using the antibody 6E10. In all figures, β-Action levels are shown as equal-loading controls. DAPT (1 µM for 16 hours), a well-known γ-secretase inhibitor, was used as a control for the accumulation of APP-C99 and APP-C83. APP-C99 and APP-C83 overexpressed in Chinese Hamster Ovary (CHO) cells are shown as controls. PMA (1 µM for 16 hours) was used as a control for increased sAPPα production. (c) Secreted Aβ X-40 were measured by ELISA (means +/-SDs, n = 3). Note that increased levels of APP-C83 and sAPPα are associated with decreased production of Aβ peptides. Also, cells exposed to a combination of Memantine and D-Serine show a large decrease in the levels of Aβ X-40 when compared to those exposed to Memantine or D-Serine alone.
Fig. 3. NMDAR modulators discriminate between the Ca2+-dependent production of APP-C99 and APP-C83. (a) The levels of intracellular APP-C99 and APP-C83 generated in the cells used in Fig. 2a were estimated by densitometry (LI-COR odyssey). (b) Densitometric estimation of secreted sAPPα shown in Fig. 2B.
Fig. 4. NMDAR modulators discriminate between the Ca2+-dependent production of APP-C99 and APP-C83. (a) Western blot analysis of intracellular APP-FL, APP-C99 and APP-C83 generated in mouse primary hippocampal neurons (15 days *in vitro*) exposed to the indicated compounds and as described in Fig. 2A. (b) sAPPα secreted in conditioned media of cultured neurons used in (a) were analyzed by Western blot using the antibody 6E10. DAPT (1 µM for 16 hours), a well-known γ-secretase inhibitor, was used as a control for the accumulation of APP-C99 and APP-C83. APP-C99 and APP-C83 overexpressed in Chinese Hamster Ovary (CHO) cells are shown as controls. PMA (1 µM for 16 hours) was used as a control for increased sAPPα production.

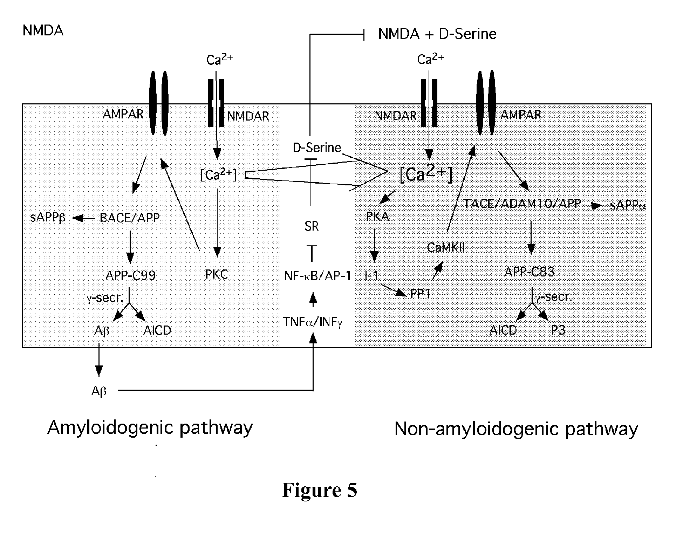

Fig. 5. Model for the NMDAR-dependent signaling pathways regulating β- and α-secretase cleavage of APP. In this model, stimulation of the NMDAR by a receptor agonist (NMDA) activates a Ca2+-dependent PKC signaling pathway which, in turn, activates the β-secretase cleavage of APP and the proteolytic production of Aβ, the amyloidogenic peptides which have been implicated in the pathogenesis of Alzheimer's disease (Amyloidogenic pathway).

Further stimulation of the NMDAR by a receptor co-agonist (D-serine) triggers an increase in the intracellular concentration of Ca2+ which, in turn, activates a Ca2+-dependent PKA- and CaMKII-dependent signaling pathway which, consequently, activates the α-secretase cleavage of APP and the non-amyloidogenic pathway. Increased Aβ levels (resulting from an activated amyloidogenic pathway) activate the production of TNFα and INFγ, which reduces the production of SR and D-serine by stimulating various transcriptions factors such as NK-kB and AP-1. Reduced D-serine levels would likely inhibit the non-amyloidogenic pathway and contribute to the development of human AD, including Aβ plaques. These two exquisitely tuned signal transduction pathways (the magnitude of the Ca2+ signal determines the cellular response), by regulating the competition between α-versus β-secretase for APP, control the complex balance between the non-amyloidogenic and amyloidogenic processing pathways. The model shown is this figure represents a hypothesis of the inventors that could explain the surprising findings reported herein. This hypothesis is not to be interpreted as a limitation of the scope of the present invention.

### Literature

1. Selkoe, D.J. Translating cell biology into therapeutic advances in Alzheimer's disease. Nature 399, A23-31 (1999).
2. Selkoe, D.J. Cell biology of the amyloid beta-protein precursor and the mechanism of Alzheimer's disease. Annu Rev Cell Biol 10, 373-403 (1994).
3. Vassar, R. & Citron, M. Abeta-generating enzymes: recent advances in beta- and gamma-secretase research. Neuron 27, 419-422 (2000).
4. Iwatsubo, T., et al. Visualization of A beta 42(43) and A beta 40 in senile plaques with end-specific A beta monoclonals: evidence that an initially deposited species is A beta 42(43). Neuron 13, 45-53 (1994).
5. Roher, A.E., et al. beta-Amyloid-(1-42) is a major component of cerebrovascular amyloid deposits: implications for the pathology of Alzheimer disease. Proc Natl Acad Sci U S A 90, 10836-10840 (1993).
6. Roher, A.E., Palmer, K.C., Yurewicz, E.C., Ball, M.J. & Greenberg, B.D. Morphological and biochemical analyses of amyloid plaque core proteins purified from Alzheimer disease brain tissue. J Neurochem 61, 1916-1926 (1993).
7. Jarrett, J.T., Berger, E.P. & Lansbury, P.T., Jr. The carboxy terminus of the beta amyloid protein is critical for the seeding of amyloid formation: implications for the pathogenesis of Alzheimer's disease. Biochemistry 32, 4693-4697 (1993).
8. Buxbaum, J.D., et al. Evidence that tumor necrosis factor alpha converting enzyme is involved in regulated alpha-secretase cleavage of the Alzheimer amyloid protein precursor. J Biol Chem 273, 27765-27767 (1998).
9. Lammich, S., et al. Constitutive and regulated alpha-secretase cleavage of Alzheimer's amyloid precursor protein by a disintegrin metalloprotease. Proc Natl Acad Sci U S A 96, 3922-3927 (1999).
10. Haass, C., Hung, A.Y., Schlossmacher, M.G., Teplow, D.B. & Selkoe, D.J. beta-Amyloid peptide and a 3-kDa fragment are derived by distinct cellular mechanisms. J Biol Chem 268, 3021-3024 (1993).
11. Parsons, C.G., Stoffler, A. & Danysz, W. Memantine: a NMDA receptor antagonist that improves memory by restoration of homeostasis in the glutamatergic system--too little activation is bad, too much is even worse. Neuropharmacology 53, 699-723 (2007).
12. Haass, C. & Selkoe, D.J. Cellular processing of β-amyloid precursor protein and the genesis of amyloid β-peptide. Cell 75, 1039-1042 (1993).
13. Skovronsky, D.M., Moore, D.B., Milla, M.E., Doms, R.W. & Lee, V.M. Protein kinase C-dependent alpha-secretase competes with beta-secretase for cleavage of amyloid-beta precursor protein in the trans-golgi network. J Biol Chem 275, 2568-2575 (2000).
14. De Strooper, B., et al. A presenilin-1-dependent gamma-secretase-like protease mediates release of Notch intracellular domain. Nature 398, 518-522 (1999).
15. Shobe, J. The role of PKA, CaMKII, and PKC in avoidance conditioning: permissive or instructive? Neurobiol Learn Mem 77, 291-312 (2002).
16. Jolly-Tornetta, C. & Wolf, B.A. Protein kinase C regulation of intracellular and cell surface amyloid precursor protein (APP) cleavage in CHO695 cells. Biochemistry 39, 15282-15290 (2000).
17. Oddo, S., et al. Triple-transgenic model of Alzheimer's disease with plaques and tangles: intracellular Abeta and synaptic dysfunction. Neuron 39, 409-421 (2003).
18. Smith, I.F., Hitt, B., Green, K.N., Oddo, S. & LaFerla, F.M. Enhanced caffeine-induced Ca2+ release in the 3xTg-AD mouse model of Alzheimer's disease. J Neurochem 94, 1711-1718 (2005).
19. Stutzmann, G.E., et al. Enhanced ryanodine receptor recruitment contributes to Ca2+ disruptions in young, adult, and aged Alzheimer's disease mice. J Neurosci 26, 5180-5189 (2006).
20. Stutzmann, G.E., et al. Enhanced ryanodine-mediated calcium release in mutant PS1-expressing Alzheimer's mouse models. Ann N Y Acad Sci 1097, 265-277 (2007).
21. Wang, Q., Wu, J., Rowan, M.J. & Anwyl, R. Beta-amyloid inhibition of long-term potentiation is mediated via tumor necrosis factor. Eur J Neurosci 22, 2827-2832 (2005).
22. Medeiros, R., et al. Connecting TNF-alpha signaling pathways to iNOS expression in a mouse model of Alzheimer's disease: relevance for the behavioral and synaptic deficits induced by amyloid beta protein. J Neurosci 27, 5394-5404 (2007).
23. Beg, A.A., Finco, T.S., Nantermet, P.V. & Baldwin, A.S., Jr. Tumor necrosis factor and interleukin-1 lead to phosphorylation and loss of I kappa B alpha: a mechanism for NF-kappa B activation. Mol Cell Biol 13, 3301-3310 (1993).
24. Angel, P. & Karin, M. The role of Jun, Fos and the AP-1 complex in cell-proliferation and transformation. Biochim Biophys Acta 1072, 129-157 (1991).
25. Akama, K.T., Albanese, C., Pestell, R.G. & Van Eldik, L.J. Amyloid beta-peptide stimulates nitric oxide production in astrocytes through an NFkappaB-dependent mechanism. Proc Natl Acad Sci U S A 95, 5795-5800 (1998).
26. Hashimoto, K., et al. Possible role of D-serine in the pathophysiology of Alzheimer's disease. Prog Neuropsychopharmacol Biol Psychiatry 28, 385-388 (2004).
27. Steiner, P., et al. Overexpression of neuronal Sec1 enhances axonal branching in hippocampal neurons. Neuroscience 113, 893-905 (2002).
28. Wiltfang, J., et al. Elevation of beta-amyloid peptide 2-42 in sporadic and familial Alzheimer's disease and its generation in PS1 knockout cells. J Biol Chem 276, 42645-42657 (2001).

## Claims

1. A pharmaceutical composition comprising an inhibitor and a co-agonist of an N-methyl-D-aspartate-type glutamate receptor (NMDAR).

2. The composition of claim 1 for use as a medicament.

3. The composition of any one of claims 1 or 2 for the prevention and/or treatment of Alzheimer's disease.

4. The composition of any one of the preceding claims, wherein the inhibitor of the NMDA receptor is a 1-aminocyclohexane or a derivative of a 1-aminocyclohexane.

5. The composition of any one of the preceding claims, wherein the inhibitor of the NMDA receptor is selected from the group of Mg²⁺, memantine (1-amino-3,5-dimethyladamantane), neramexane (1-amino-1,3,3,5,5-pentamethylcyclohexane), 1-aminocyclohexane, phencyclidine, MK-801 ((+)-5-methyl-10,11- dihydro-5H-dibenzo[a,d]cyclohepten-5,10-imine maleate), ARL12495AA ((*S*)--phenyl-2-pyridine-ethanamine dihydrochloride) and ketamine (2-(2-chlorophenyl)-2-methylamino-cyclohexan-1-one), variants and/or derivatives of the aforementioned including pharmaceutically acceptable salts, optical isomers, diastereomers, polymorphs, hydrates and combinations of two or more of the aforementioned compounds.

6. The composition of any one of the preceding claims, wherein the inhibitor of the NMDA receptor is selected from competitive antagonists and NR2B antagonists selected from CPP (3-(2-Carboxypiperazin-4-yl)propyl-1-phosphonic acid), AP5 (2-amino-5-phosphonovalerate), CGS19755 (cis-4-[Phosphomethyl]-piperidine-2-carboxylic acid), LY235959 (3S-(3a,4aa,6b,8aa)]-Decahydro-6-(phosphonomethyl)-3-is oquinolinecarboxylic acid), LY233053 ((2R*,4S*)-4-(1H-Tetrazol-5-ylmethyl)-2-piperidinecarbox ylic acid), Ro25-6981 ((aR,bS)-a-(4-Hydroxyphenyl)-b-methyl-4-(phenylmethyl)-1 -piperidinepropanol maleate), CP101606 (taxoprodil; (1S,2S)-1-(4-hydroxyphenyl)-2-(4-hydroxy-4-phenylpiperidino)-1-propanol),), CI1041(6-((2-(4-(4-fluorophenyl)methyl)-1-piperidinyl)ethyl)sulfinyl)-2-(3H)-benzoxazolone), ifenprodil ((1R*,2S*)-erythro-2-(4-Benzylpiperidino)-1-(4-hydroxyphenyl)-1-propanol), variants and/or derivatives of the aforementioned, and combinations of two or more of the aforementioned compounds.

7. The composition of any one of the preceding claims, wherein the 1-aminocyclohexane or its derivative is a compound of formula (I): 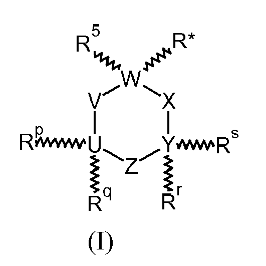 wherein:
R* is -(A)n-(CR¹R²)m-NR³R⁴,
n and m are integers, and n+m=0, 1, or 2,
A is selected from the group consisting of linear or branched lower alkyl (C1-C6), linear or branched lower alkenyl (C2-C6), and linear or branched lower alkynyl (C2-C6);
R¹ and R² are independently selected from the group consisting of hydrogen, linear or branched lower alkyl (C1-C6), linear or branched lower alkenyl (C2-C6), linear or branched lower alkynyl (C2-C6) aryl, substituted aryl and arylalkyl;
R³ and R⁴ are independently selected from the group consisting of hydrogen, linear or branched lower alkyl (C1-C6), linear or branched lower alkenyl (C2-C6), and linear or branched lower alkynyl (C2-C6), or together form alkylene (C2-C10) or alkenylene (C2-C10) or together with the N form a 3-7-membered azacycloalkane or azacycloalkene, including a substituted (alkyl (C1-C6), alkenyl (C2-C6)) 3-7-membered azacycloalkane or azacycloalkene; or independently R³ or R⁴ may join with R^{p}, R^{q}, R^{r}, or R^{s} to form an alkylene chain -CH(R⁶)-(CH₂)t-, wherein t=0 or 1 and the left side of the alkylene chain is attached to U or Y and the right side of the alkylene chain is attached to N and R⁶ is selected from the group consisting of hydrogen, linear or branched lower alkyl (C1-C6), linear or branched lower alkenyl (C2-C6), linear or branched lower alkynyl (C2-C6), aryl, substituted aryl and arylalkyl; or independently R³ or R⁴ may join with R⁵ to form an alkylene chain represented by the formula -CH2-CH2-CH2-(CH2)t-, or an alkenylene chain represented by the formulae -CH-CH-CH2-(CH2)t-, -CH-C-CH-(CH2)t- or -CH2-CH-CH-(CH₂)ₜ-, wherein t=0 or 1, and the left side of the alkylene or alkenylene chain is attached to W and the right side of the alkylene ring is attached to N;
R⁵ is independently selected from the group consisting of hydrogen, linear or branched lower alkyl (C1-C6), linear or branched lower alkenyl (C2-C6), and linear or branched lower alkynyl (C2-C6), or R⁵ combines with the carbon to which it is attached and the next adjacent ring carbon to form a double bond;
R^{p}, R^{q}, R^{r}, and R^{s}, are independently selected from the group consisting of hydrogen, linear or branched lower alkyl (C1-C6), linear or branched lower alkenyl (C2-C6), linear or branched lower alkynyl (C2-C6), cycloalkyl (C3-C6) and aryl, substituted aryl and arylalkyl or R^{p}, R^{q}, R^{r}, and R^{s} independently may form a double bond with U or with Y or to which it is attached, or R^{p}, R^{q}, R^{r}, and R^{s} may combine together to represent a lower alkylene -(CH₂)ₓ-or a lower alkenylene bridge wherein x is 2-5, inclusive, which alkylene bridge may, in turn, combine with R⁵ to form an additional lower alkylene -(CH₂)_{y}- or a lower alkenylene bridge, wherein y is 1-3, inclusive; and
the ring defined by U-V-W-X-Y-Z represents an optionally unsaturated cyclohexane ring wherein U, W, and Y represent carbon atoms and V, X, and Z each independently represent a carbon atom, CH, or CH₂, (or the definitions of U, W, Y on one hand and V, X, and Z can be reversed including corresponding placement of the R groups R*, R⁵, R^{p}, R^{q}, R^{r}, and R^{s}), it being understood that the valence requirements of the ring atoms are respected, and include optical isomers, diastereomers, polymorphs, enantiomers, hydrates, pharmaceutically acceptable salts, and mixtures of compounds within formula (I).

8. The composition of any one of the preceding claims, wherein the co-agonist of the NMDAR is selected from D-alanine, D-cycloserine, D-serine, N-methylglycine, D-phosphoserine, L-phosphoserine, glycine, derivatives and/or variants of the aforementioned, and combinations comprising two or more of the aforementioned compounds.

9. The composition of any one of the preceding claims, wherein the NMDAR antagonist is memantine or an active derivative of memantine.

10. The composition of any one of the preceding claims, wherein the NMDAR co-agonist is D-serine.

11. The composition of any one of the preceding claims, in which the NMDAR inhibitor and the NMDAR antagonist are provided separately, preferably in the form of separate administration units.

12. An inhibitor and a co-agonist of the NMDAR for use as a medicament.

13. The inhibitor and co-agonist of NMDAR of claim 12, which are administered simultaneously or sequentially.

14. A method of preventing and/or treating Alzheimer's disease, the method comprising the step of administering to a person in need thereof a pharmaceutically effective amount of an inhibitor and a co-agonist of the N-methyl-D-aspartate-type glutamate receptor (NMDAR).

15. the method of claim 11, wherein the step of administering the antagonist and the coagonist of the NMDRA, these compounds are administered sequentially.
